# EUROPEAN PATENT SPECIFICATION

(11) **EP 4 262 603 B1**
(45) Date of publication and mention of the grant of the patent: **10.12.2025**
(21) Application number: 21836644.1
(22) Date of filing: 20.12.2021
(51) Int. Cl.: A61B 18/22

(54) **APPARATUS FOR LASER MORCELLATION**
VORRICHTUNG ZUR LASER-MORCELLATION
APPAREIL DE MORCELLATION LASER

(30) Priority: 20.12.2020 US 202063128138 P
(43) Date of publication of application: 25.10.2023
(60) Divisional application: 25208261.5
(73) Proprietor: Lumenis Ltd., 2069204 Yokneam (IL)
(72) Inventor: ROSEN, Silvio, 2069204 Yokneam (IL); KHACHATUROV, Arkady, 2069204 Yokneam (IL)
(74) Representative: Vossius & Partner Patentanwälte Rechtsanwälte mbB
(86) International application number: PCT/IB2021/062049
(87) International publication number: WO 2022/130362

(56) References cited:
- WO-A1-91/06271
- WO-A2-2020/150713
- US-A- 4 694 828
- US-A- 6 024 751
- US-A1- 2002 128 637
- US-A1- 2004 158 236

## Description

### Related Applications

This application claims the benefit of priority under 35 U.S.C. §119 to U.S. Provisional Patent Application No. 63/128,117, titled "Tip for a Morcellation Apparatus", filed on December 20, 2020.

### Technical Field

The present disclosure generally relates to the field of tissue removal. Particularly, but not exclusively, the present disclosure relates to a tip for a laser apparatus for dissecting and extracting tissue from the body.

### Background

Introduction of lasers into medical field and development of fiber optic technologies that use lasers have opened wide range of applications in treatments, diagnostics, therapies, and the like. Such applications range from invasive and non-invasive treatments to endoscopic surgeries and image diagnostics. For example, lasers are used to treat diseases of the prostate.

The prostate is a relatively small gland located inside the groin of males underneath the bladder. The prostate gland is located between the base of the penis and the rectum. The prostate is a male reproductive gland that surrounds the lower portion of the bladder where urine is stored and part of the urethra through which urine passes from the bladder out of the body. Benign Prostatic Hypertrophy (BPH) is a condition in which the prostate gland is enlarged by over proliferation of the smooth muscles and epithelial cells. This enlargement causes squeezing of the lower portion of the bladder and the urethra. As a result, for these patients, it is difficult to pass urine. This and additional symptoms are expressed and defined as "lower urinary tract syndrome" (LUTS). To treat LUTS, surgical removal of the gland is often performed. The excess prostatic tissue (adenoma) is removed from the interior region of the prostate (capsule) that is pressing on the urethra, which usually relieves the obstruction and the incomplete emptying of the bladder caused by the BPH condition. The rest of the prostatic tissue is typically left intact. Surgeons can perform enucleation surgery to remove the excess prostate tissue through the urethra, or transurethral. For example, a surgeon can insert a resectoscope through the urethra. The resectoscope is used to view the interior of the urinary tract, and to cut off pieces of the targeted prostatic tissue into the bladder. The enucleated/resected off tissue of the prostate is then dissected into small enough chunks suitable for subsequent extraction from the bladder.

There are several prostate resection procedures in use. One is holmium enucleation of the prostate (HoLEP), in which prostatic tissue is morcellated and enucleated with laser energy and the cut-up tissue pushed into the bladder. The prostatic tissue in the bladder is further mechanically morcellated into several smaller chunks suitable for extraction from the body. Conventional HoLEP procedures require the use of two different apparatuses. The first apparatus is a laser, used for enucleation of the prostate tissue, which is delivered with an optical fiber through a cystoscope; while the second apparatus is a morcellator, used for morcellation of the enucleated tissue, which is inserted into the bladder through a nephroscope.

This process of using two different apparatuses for the removal of prostate tissue is time consuming and involves risks and challenges that can be improved with the present invention that aims to replace the need for the mechanical morcellation. For example, mechanical morcellation makes use of moving blades for cutting the prostate tissue. The pieces of prostatic tissue cut by the blades must be small enough to be sucked out through the blade shafts. The process of morcellation has several risks associated with it, with the main risk being perforation of the bladder wall. In addition, since there may be many excised pieces of prostatic tissue, subsequent remove of the cut-up tissue from the bladder can be time consuming.

WO 2020/0150713 A2 discusses an instrument for endoscopic applications, including urology. The instrument may include both irrigation and aspiration channels, effective attraction and suction of tissue and body stone fragments, enhanced viewing clarity of the operational area, illumination fibers with steering function for flexible version of the scopes. In some embodiments, a distal head is configured to locate a mouth of the working channel within a viewing angle of the visualization system. In some embodiments, a transparent cap is disposed at the distal end of endoscope to provide an enhanced view of the operational area. Irrigation and aspiration channels may be arranged so that consistent water flow will attract tissue and body stone particles and remove heated liquid. Illumination fibers may be utilized as pull linkages or push-pull linkages for deflection and steering of flexible embodiments of the scope.

### Brief Summary

The invention is defined in the appended set of claims. In an embodiment, the present disclosure provides an apparatus for morcellating and removing targeted body tissue of a subject, such as, for example, prostatic tissue. The apparatus includes a holder defining a housing with an elongated cannula defining a fiber tube and a suction tube. One end of the cannula is coupled to the holder and the other end of the cannula is configured to accommodate a plug. The plug includes a body defined with an inclined tip surface. A first aperture is defined along a longitudinal axis of the body and is configured to accommodate an optical fiber. A second aperture is configured adjacent to the first aperture and is arranged to be fluidly connectable to a suction tube. The longitudinal axis of the first aperture and the inclined tip surface can be oriented at a pre-determined angle. The optical fiber is configured to transmit a laser beam for dissecting the targeted body tissue into smaller chunks of tissue such that the smaller chunks of the tissue are removed through the suction tube.

In another embodiment, the present disclosure provides a tip attached to the distal (front or working) end of a morcellation apparatus. The tip includes a body defined with an inclined tip surface. A first aperture is defined along a longitudinal axis of the body and is configured to accommodate an optical fiber. A second aperture is configured adjacent to the first aperture and is arranged to be fluidly connectable to a suction tube. Further, the longitudinal axis of the first aperture and the inclined tip surface can be oriented at a pre-determined angle.

The invention may be used in a method for morcellating a targeted tissue of a subject. The method includes introducing an elongated cannula defined with a fiber tube and a suction tube into a urethra, where one end of the cannula is removably coupled to the holder and the other end of the cannula is configured with a removable tip defined with an inclined tip surface. The tip includes a first aperture defined along a longitudinal axis of the body configured to accommodate the fiber tube with an optical fiber. A second aperture is configured adjacent to the first aperture and is fluidly connected to the suction tube. Further, the longitudinal axis of the first aperture and the inclined tip surface are oriented at a pre-determined angle. The method further includes guiding the elongated cannula, by a user, to the targeted tissue and activating a laser source coupled to the optical fiber housed in the fiber tube to transmit a laser beam through the optical fiber for morcellating the targeted body tissue into smaller chunks of tissue. A vacuum source fluidically connected to the suction tube is operated, simultaneously with or after activation of the laser source, for removing the dissected chunks of tissues.

### Brief Description of the Drawings

Non-limiting embodiments of the present disclosure are described by way of example with reference to the accompanying figures, which are schematic and not intended to be drawn to scale. In the figures, each identical or nearly identical component illustrated is typically represented by a single numeral. In will be appreciated that various figures included in this disclosure may omit some components, illustrate portions of some components, and/or present some components as transparent to facilitate illustration and description of components that may otherwise appear hidden. For purposes of clarity, not every component is labelled in every figure, nor is every component of each embodiment shown where illustration is not necessary to allow those of ordinary skill in the art to understand the disclosure. In the figures:
**FIG. 1A** and **FIG. 1B** illustrate a tissue dissecting apparatus, in accordance with some embodiments of the disclosure.
**FIG. 2** illustrates a handpiece for a tissue dissecting apparatus, in accordance with some embodiments of the disclosure.
**FIG. 3A, FIG. 3B,** and **FIG. 3C** illustrate perspective view and side views of a tip for a tissue dissecting apparatus, in accordance with some embodiments of the disclosure.
**FIG. 4A, FIG. 4B****,** **FIG. 4C,** and **FIG. 4D** illustrates perspective views of tips for a tissue dissecting apparatus, in accordance with some embodiments of the disclosure.
**FIG. 5A, FIG. 5B,** and **FIG. 5C** illustrate side views of a tip for a tissue dissecting apparatus, in accordance with some embodiments of the disclosure.
**FIG. 6****,** illustrates a front view of a tip for a tissue dissecting apparatus, in accordance with some embodiments of the disclosure.
**FIG. 7A, FIG. 7B,** and **FIG. 7C** illustrate side views of the tip for the tissue dissecting apparatus of **FIG. 6** in alternative configurations, in accordance with some embodiments of the disclosure.
**FIG. 8A** and **FIG. 8B** illustrate perspective and side views of a tip for tissue dissecting apparatus, in accordance with some embodiments of the disclosure.
**FIG. 9A** and **FIG. 9B** illustrate a cannula for a tissue dissecting apparatus, in accordance with some embodiments of the disclosure.
**FIG. 9C** illustrates a tip for a tissue dissecting apparatus for the cannula of **FIG. 9A** and **FIG. 9B****,** in accordance with some embodiments of the disclosure.
**FIG. 10A** and **FIG. 10B** illustrate a cannula for a tissue dissecting apparatus, in accordance with some embodiments of the disclosure.
**FIG. 10C** illustrates a tip for a tissue dissecting apparatus for the cannula of **FIG. 10A** and **FIG. 10B****,** in accordance with some embodiments of the disclosure.
**FIG. 11** illustrates a tip for a tissue dissecting apparatus, in accordance with some embodiments of the disclosure.
**FIG. 12** illustrates a tissue dissecting system, in accordance with some embodiments of the disclosure.

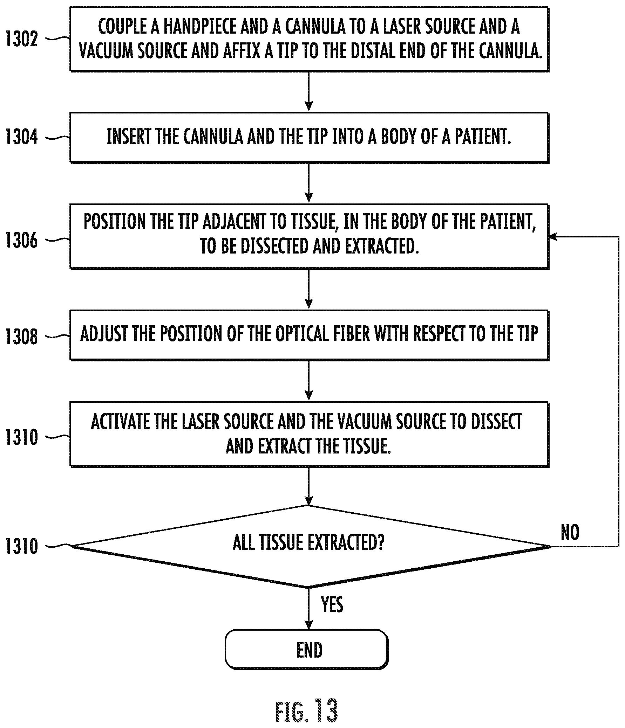

**FIG. 13** illustrates a routine for dissecting and extracting tissue, in accordance with some embodiments of the disclosure.

### Detailed Description

As outlined above, the present disclosure provides an apparatus for morcellating and removing targeted body tissue of a subject, including embodiments that provide a tip for applying laser morcellation and tissue removal. Many of the examples described herein use prostatic tissue and are described in the context of prostate enucleation. However, the present disclosure can be applied to laser morcellation and tissue removal for any tissue in the body. Additionally, the present disclosure can be utilized with any of a variety of laser sources, such as holmium, thulium, or the like.

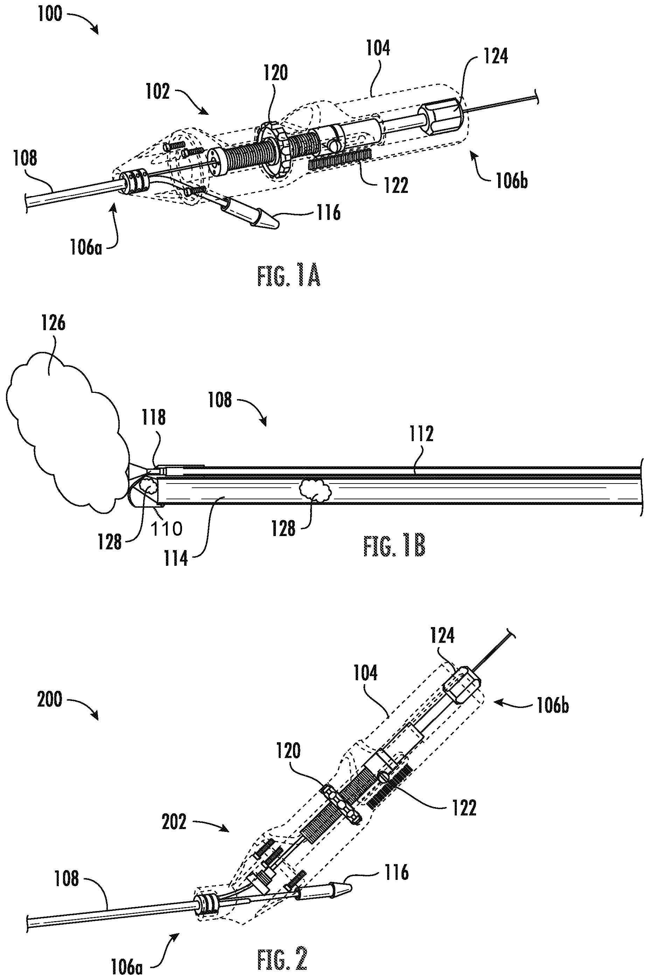

**FIG. 1A** and **FIG. 1B** illustrate a tissue dissection apparatus 100. Tissue dissection apparatus 100 comprises a handpiece 102, also referred to as a holder, which defines a housing 104. In general, handpiece 102 is configured with a shape suitable for grasping or gripping by a user (e.g., medical practitioner, physician, technician, or the like) and for ease of manipulation or maneuverability and to be firmly held in the hands of a medical practitioner.

The handpiece 102 is defined by a front (or distal) end 106a and a rear (or proximal) end 106b. The distal end 106a of the handpiece 102 may define a cavity for accepting a cannula 108. In some embodiments, the cannula 108 can be removably coupled to the handpiece 102. One end of the cannula 108 may be configured to removably couple to a tip 110. With some embodiments, the cannula 108 comprises a fiber tube 112 and a suction tube 114. Suction tube 114 may be fluidly coupled to a vacuum source via connection port 116 at housing 104 of the handpiece 102.

The fiber tube 112 of the cannula 108 is configured to house an optical fiber 118. In some embodiments, the optical fiber 118 can extend from the proximal end 106b of the handpiece 102 into the fiber tube 112 of the cannula 108 coupled at the distal end 106a of the handpiece 102, where the optical fiber is coupled to a laser source (not shown). The handpiece 102 may comprise (e.g., in housing 104, or the like) an optical fiber adjustment unit 120, a position indication unit 122, and a locking unit 124. The fiber adjustment unit 120 may include one or more rollers (not labeled) which may press on, or put pressure on, the optical fiber 118. Movement of the rollers may cause the optical fiber 118 to advance in a distal direction or retract in a proximal direction. The optical fiber 118 extending through handpiece 102 may also extend through the optical fiber adjustment unit 120, the position indication unit 122 and the locking unit 124. In some embodiments, the locking unit 124 may be placed at the proximal end 106b of the handpiece 102. The indication unit 122 and the optical fiber adjustment unit 120 may be placed along a central region of the handpiece 102.

With some embodiments, the optical fiber adjustment unit 120 may include a linear bearing coupled to a roller and the linear bearing may further be coupled to the position indication unit 122. The medical practitioner who desires to vary the position of the optical fiber 118 may rotate the roller in a clockwise or an anti-clockwise direction and the optical fiber adjustment unit 120 may translate this rotational motion of the roller into a forward (e.g., distal) or backward (e.g., proximal) movement of the optical fiber 118. The roller and the linear bearing may be configured such that the clockwise rotation of the roller results in forward movement of the linear bearing and the anti-clockwise directional rotation of the roller results in backward movement of the roller or vice versa.

With some embodiments, the optical fiber adjustment unit 120 can include a roller arranged perpendicularly to the roller depicted in FIG. 1A (e.g., the roller can be arranged in the same direction as the fiber). As such, rotating the roller forward (e.g., in the distal direction) will adjust the fiber forward or towards the front end 106a while rotating the roller backwards (e.g., in the proximal direction) will adjust the fiber backwards or towards the proximal end 106b.

Further, the optical fiber adjustment unit 120 may be coupled to the position indication unit 122 and a quantity of forward or backward movement of the optical fiber 118 responsive to actuation of the optical fiber adjustment unit 120 may be indicated in by position indication unit 122. The position indication unit 122 indicating the position of the optical fiber 118 in the fiber tube 112 may provide suitable feedback to the clinician, who may further adjust the position of the optical fiber 118 relative to the cannula 108 with the fiber adjustment unit 120.

In some embodiments, the locking unit 124 in the handpiece 102 is arranged to secure or "fix" the position of the optical fiber 118 relative to the cannula 108. The locking unit 124 may initially be operated into an un-locked condition and the position of the optical fiber 118 may be adjusted by the optical fiber adjustment unit 120. Once the position of the optical fiber 118 has been suitably adjusted, the locking unit 124 may be actuated or placed into a locked condition and the position of the optical fiber 118 may thereby be fixedly secured.

During use, the cannula 108 with the tip 110 attached (or mounted) on the distal end of the cannula 108 may be inserted into the body to remove tissue 126. For example, where tissue 126 to be morcellated and excised by tissue dissecting apparatus 100 is prostate tissue that has been cut and pushed into the bladder or a patient, the cannula 108 can be inserted into the bladder of the patient (or subject) through the urethra of the patient. The cannula 108 can be maneuvered to be positioned adjacent to the tissue 126 inside the bladder. This tissue 126 is then further morcellated or incised into smaller pieces and removed from the bladder by the tissue dissecting apparatus 100. More specifically, the tissue 126 is further morcellated by laser energy delivered from the laser source to the tissue 126 via the optical fiber 118 while the tissue 126 is removed from the body by vacuum or suction delivered from the vacuum source via the suction tube 114.

The vacuum source connected to the suction tube 114 and the laser source connected to the optical fiber 118 may be simultaneously operated. During operation, tissue 126 is initially anchored to the distal end of the tip 110 due to the suction created in the suction tube 114. The medical practitioner may vary the position of the optical fiber 118 inside the fiber tube 112 and may maneuver the cannula 108 such that a piece of the tissue 126 is dissected (or cut) into smaller pieces 128. The dissected pieces 128 of tissue 126 may then be sucked into the suction tube 114 and thereby extracted from the body.

This process of cutting the tissue 126 into smaller pieces 128 and simultaneously extracting the smaller pieces 128 of the tissue 126 from the body through the suction tube 114 may be repeated multiple of times until the tissue 126 is dissected and removed from the body. For example, during a normal enucleation procedure to treat BHP, multiple pieces of tissue 126 may be in the bladder to be dissected and extracted as described herein.

In some embodiments, a single tissue dissecting apparatus 100 may be used for enucleation and dissection. For example, a BPH treatment procedure can include inserting the tissue dissecting apparatus 100 into the bladder through the urethra of the body of the patient. Subsequently, prostate tissue 126 may be enucleated or cut with laser energy delivered via the optical fiber 118. Further, the same tissue dissecting apparatus 100 and the same optical fiber 118 may be used for dissecting and extracting the enucleated and cut tissue via laser energy and vacuum. Accordingly, the present disclosure provides an advantage in that a single device can be used to both enucleation and dissection and removal of prostate tissue. Additionally, the present disclosure provides several embodiments and configuration for the tip 110 connected to the distal end of the cannula 108, which have various advantages, such as, prevention of clogging the suction tube 114.

**FIG. 2** illustrates an embodiment of a tissue dissection apparatus 200, which is like the tissue dissecting apparatus 100 described above. However, tissue dissection apparatus 200 includes a handpiece (or holder) 202 that is different from the handpiece or holder 102. As can be seen, the handpiece 202 is curved such that the suction tube 114 continues straight through the handpiece 202 to the connection port 116 while the optical fiber 118 is curved whereas the suction tube 114 is curved in the handpiece 102 while the optical fiber 118 is straight. In some embodiments, the straight suction tube hose shown in FIG.2 may have an advantage over the curved suction tube shown in FIG. 1A. For example, a straight suction tube configuration has an advantage in that increased water and/or tissue removal flow rates as well as a reduction in clogging.

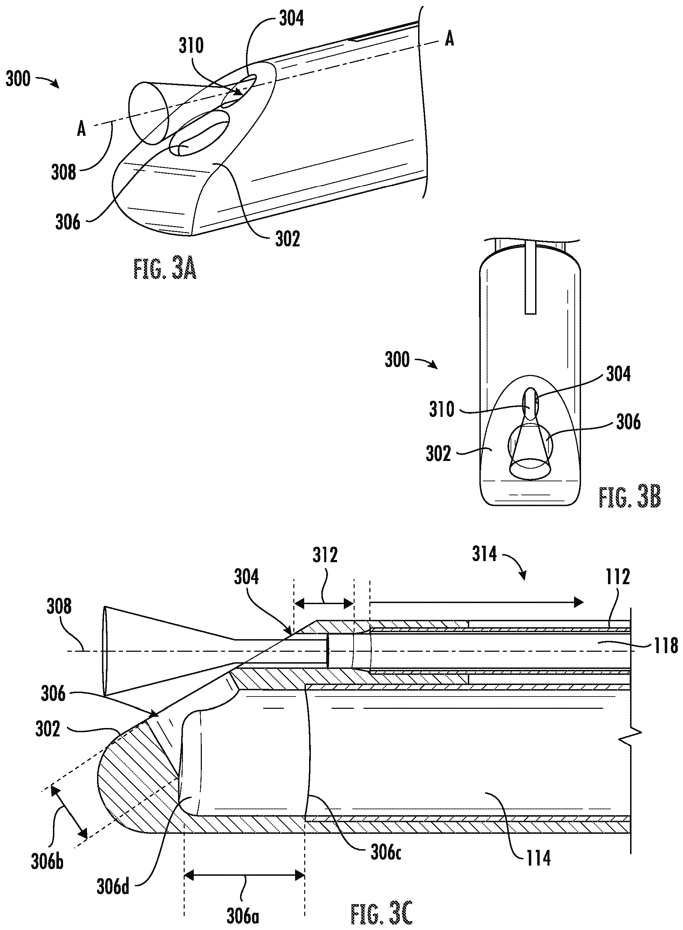

**FIG. 3A, FIG. 3B,** and **FIG. 3C** illustrate perspective and side views of a tip 300 for a tissue dissecting apparatus. In general, the tip 300 can be the provided as the tip 110 of the tissue dissecting apparatus 100 or 200. In some embodiments, the tip 300 is comprised of a metallic material with a high melting temperature to provide heat resistance to the heat or energy radiated by optical fiber 118. For example, the tip 300 can comprise tungsten, which has a melting temperature of about 3420 degrees Celsius and a tensile strength of about 2500 Megapascals (MPa). In other operations, the tip 300 can comprise stainless steel. Additionally, it is noted that an advantage of the present disclosure and the below claims is that the distance of the fiber tip from the tip 300 is managed such that the tip 300 does not absorb significant amounts of energy from laser emissions. In some embodiments, the tip 300 may be disposable after each use. The tip 300 may be defined with an inclined tip surface 302 defined along a front or distal edge of the tip 300. Additionally, two apertures 304 and 306 are defined in the inclined surface 302. The apertures 304 and 306 may traverse a central length of the tip 300.

For example, the first aperture 304 may be defined along a longitudinal axis 308 (A-A) of the tip 110 while the second aperture 306 may be defined along the longitudinal axis 308 of the tip 110 adjacent to the first aperture 304. In some embodiments, the second aperture 306 may be defined below the first aperture 304.

The aperture 304 is arranged to accept or accommodate the fiber tube 112 of the cannula 108, which itself houses the optical fiber 118. The aperture 304 may be of uniform or a varying diameter. In some embodiments, the aperture 304 is defined with a first diameter along a first length 312 of the longitudinal axis 308 from the inclined tip surface 302 (or the entrance to the aperture 304), followed by a second diameter, that is larger than the first diameter, for a second length 314 (or the remaining length, etc.) along the longitudinal axis 308. The aperture 304 may be configured such that the second diameter 314 extends after the first diameter 312 ends. The portion of the aperture 304 with the larger diameter extending along length 314 may accommodate the fiber tube 112 of the cannula 108 while the portion of the aperture 304 with the smaller diameter extending along length 312 may accommodate the optical fiber 118 of the cannula 108. In such a manner, the optical fiber 118 can be adjusted (e.g., via the optical fiber adjustment unit 120, or the like) to be flush with, recessed slightly into, or extend out from the inclined tip surface 302 such that when the laser source is activated laser energy 310 can be emitted from the tip 300.

The aperture 306 includes a first portion 306a and a second portion 306b where, the first portion 306a of the aperture 306 is defined by a first end 306c and a second end 306d. The first portion 30a of the aperture 306 extends in a direction parallel to the longitudinal axis 308 of the aperture 304 and the second portion 306b of the aperture 306 may be configured to extend in a direction that is substantially perpendicular to the inclined tip surface 302. The second portion 306b of the aperture 306 is configured such that the inclined tip surface 302 is defined with a circular opening to form the second aperture 306.

The portion 306b of the aperture 306 is fluidically connected to the second end 306d of the first portion 306a. The first end 306c of the first portion 306a in the aperture 306 is fluidly connectable to the suction tube 114 of the cannula 108. The first end 306c also forms a shoulder stopper that fixes the distal end of the suction tube 114 within the tip 300. Further, in some embodiments, the tip 300 is configured with a pre-determined orientation angle between the inclined tip surface 302 and the longitudinal axis 308 of the aperture 304.

The apertures 304 and 306 can be defined with a variety of geometric shaped openings, such as, for example, circular, oval, or the like. Additionally, the openings of apertures 304 and 306 can be defined by different geometric shapes, for example, the aperture 304 can define a circular aperture while the aperture 306 can define an oval aperture.

It is to be appreciated, that the angular orientation between the longitudinal axis 308 of the aperture 304 housing the optical fiber 118 and the inclined tip surface 302 of the tip 300 prevents the clogging of tissue in the suction tube 114. The circular cross-section defined on the inclined tip surface 302 of the tip 300 along with the configuration of the first portion 306a and second portion 306b of the second aperture 306 also reduces the clogging of tissue in the suction tube 114.

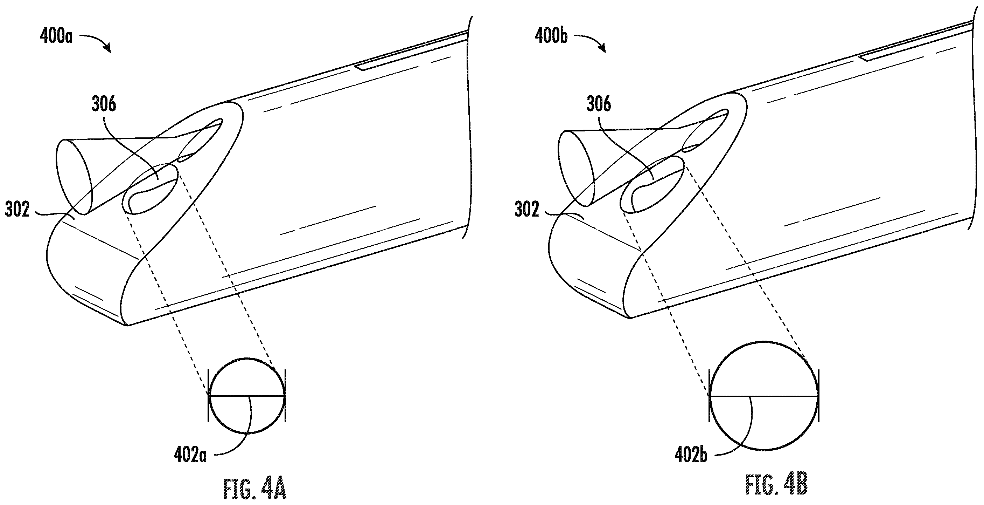

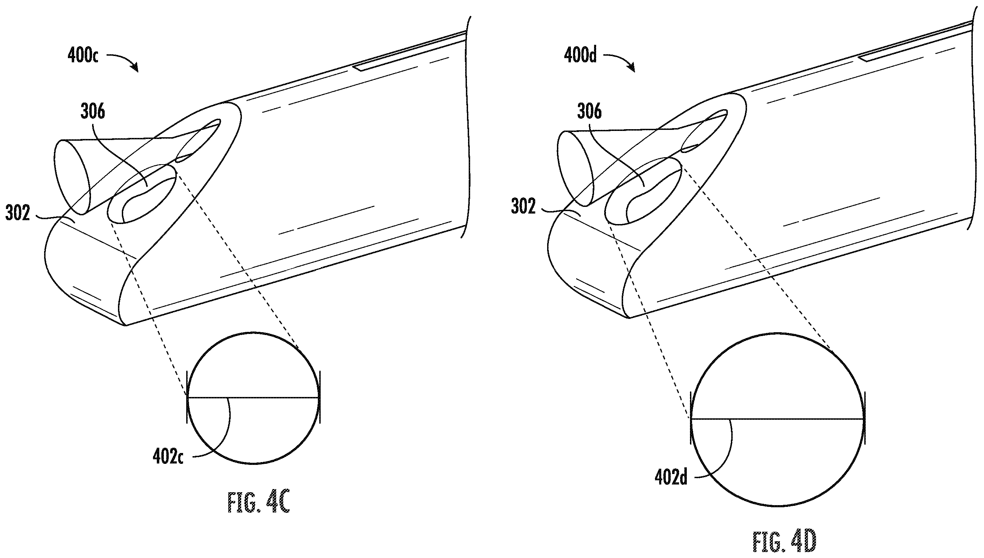

In general, the opening defined by the aperture 306 can be between 1.8 millimeters (mm) and 2.5 mm in diameter. For example, **FIG. 4A, FIG. 4B****,** **FIG. 4C,** and **FIG. 4D** illustrates tips 400a, 400b, 400c, and 400d, respectively, for a tissue dissecting apparatus. In general, the tips depicted in these figures can be like the tip 300 or can include features described with respect to tip 300 and can be provided as the tip 110 of the tissue dissecting apparatus 100 or 200. The tips each have a second aperture 306 which defines an opening in the inclined tip surface 302 having a diameter between 1.8 mm and 2.5 mm.

**FIG. 4A** depicts the tip 400a with aperture 306 defining an opening in the inclined tip surface 302 comprising a diameter 402a of 1.8 mm; **FIG. 4B** depicts the tip 400b with aperture 306 defining an opening in the inclined tip surface 302 comprising a diameter 402b of 2 mm; **FIG. 4C** depicts the tip 400c with aperture 306 defining an opening in the inclined tip surface 302 comprising a diameter D3c of 2.2 mm; and **FIG. 4D** depicts the tip 400d with aperture 306 defining an opening in the inclined tip surface 302 comprising a diameter D3d of 2.5 mm.

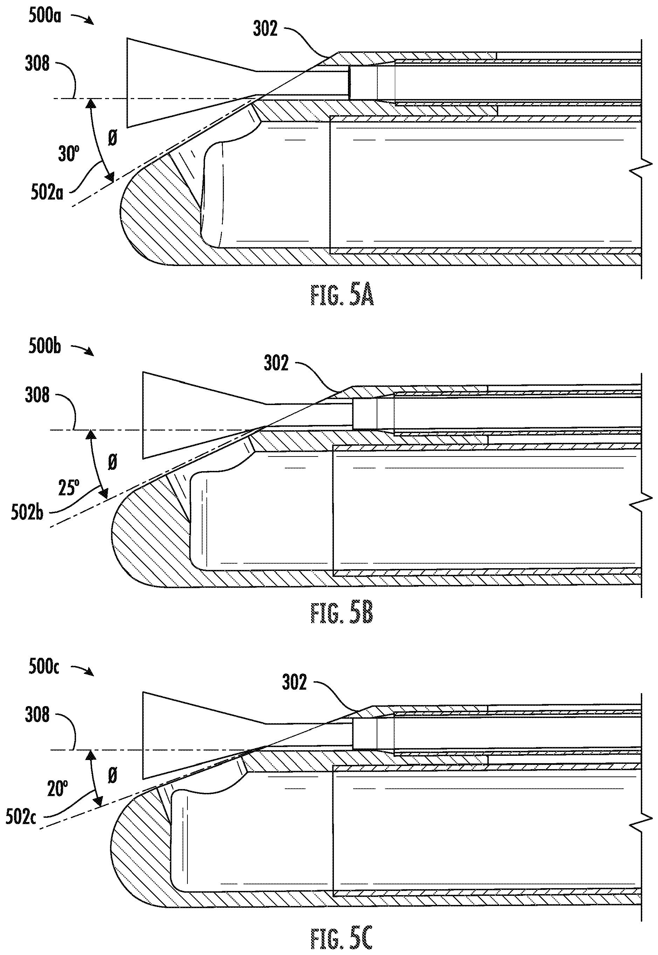

In general, the inclined tip surface 302 can be arranged to define an angle between 15 and 45 degrees with respect to the longitudinal axis 308 of the aperture 304. For example, **FIG. 5A, FIG. 5B,** and **FIG. 5C** illustrates tips 500a, 500b, and 500c, respectively, for a tissue dissecting apparatus. In general, the tips depicted in these figures can be like the other tips described herein (e.g., the 300, the tip 400a, the tip 400b, the tip 400c, or the tip 400d) or can include features described with respect to these tips and can be provided as the tip 110 of the tissue dissecting apparatus 100 or 200. The tips shown in these figures each have an inclined tip surface 302 where the angle between the inclined tip surface 302 and the longitudinal axis 308 of the aperture 304 (not labeled for clarity) is between 20 and 30 degrees.

**FIG. 5A** depicts the tip 500a with the inclined tip surface 302 defining an angle 502a with the longitudinal axis 308 of the aperture 304 of 30 degrees; **FIG. 5B** depicts the tip 500b with the inclined tip surface 302 defining an angle 502b with the longitudinal axis 308 of the aperture 304 of 30 degrees; and **FIG. 5C** depicts the tip 500c with the inclined tip surface 302 defining an angle 502c with the longitudinal axis 308 of the aperture 304 of 20 degrees.

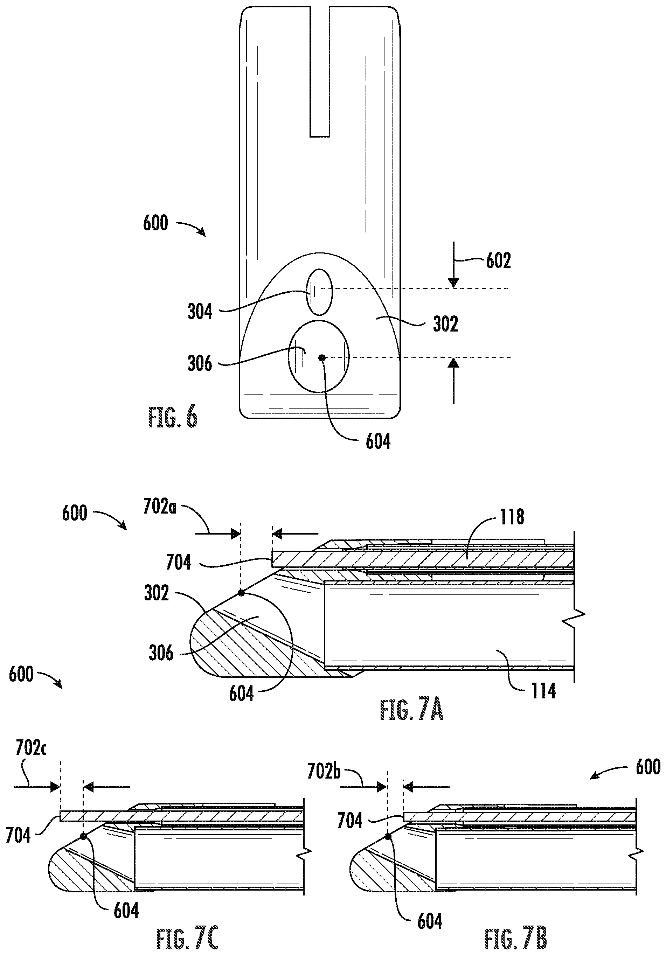

**FIG. 6** illustrates a tip 600. The tip 600 can be like the other tips described herein (e.g., the 300, the tip 400a, the tip 400b, the tip 400c. the tip 400d, the tip 500a, the tip 500b, or the tip 500c) or can include features described with respect to these tips and can be provided as the tip 110 of the tissue dissecting apparatus 100 or 200. Tip 600 shows a distance 602 defined between the longitudinal axis 308 of the aperture 304 and central point 604 of the aperture 306.

The laser energy 310 that is transmitted by the distal end of the optical fiber 118 is effective up to a certain distance. Where the distance 602 is too lengthy, the laser energy 310 may be insufficient to separate or cut the entire tissue 126. As such, in some embodiments, the distance 602 is within the range of 1 mm to 4 mm.

**FIG. 7A, FIG. 7B,** and **FIG. 7C** illustrate the tip 600 of **FIG. 6** with the optical fiber 118 disposed different distances from the point 604. These figures depict a distal tip 704 (or output facet) of optical fiber 118 extending or protruding from aperture 304. These figures depict a distance 702 between the distal tip 704 and the central point 604 of the aperture 306. The distance 702 varies as the optical fiber 118 is moved (e.g., advanced, retracted, or the like) via optical fiber adjustment unit 120. As the optical fiber 118 moves forward, the distance 702 decreases (or increases in the negative, e.g., distance 702c) while as the optical fiber 118 is retracted the distance 702 increases (or decreases in the negative, e.g., distance 702c). In some embodiments, the optical fiber adjustment unit 120 is arranged to displace the distal tip 704 between 1.2 mm and -1.2 mm from the central point 604, and preferably between 1.15 mm and negative 1.2 mm, where distance is measured along the X-axis when the tip 600 is viewed from the side as shown in these figures and where the central point 604 is at 0 on the X-axis.

It is to be appreciated that when the optical fiber 118 is retracted and the distal tip 704 comes adjacent to the body of the tip 600, sparks may be generated when laser energy is emitted from the distal tip 704 of the optical fiber 118. Additionally, the tip 600 may be heated excessively when the distal tip 704 of the optical fiber 118 is adjacent to the body of the tip 600. The heat and sparks could damage healthy tissue in the vicinity of the tissue being morcellated and extracted. Additionally, where the distance 702 is negative, the distal tip 704 of the optical fiber 118 may protrude too far away from the body of the tip 600, which may result in unwanted tissue perforation. Consequently, an effective cutting of the tissue cannot be achieved.

The position indication unit 122 can be arranged to provide feedback for a user (e.g., medical practitioner, or the like) such that the optical fiber 118 and particularly the distal tip 704 can be adjusted to within the desired range. As another example, position indication unit 122 can be disposed adjacent to another viewing apparatus (e.g., endoscope display, or the like) or can be integrated into a graphical user interface such that the user (e.g., medical practicioner, or the like) can see the tissue or site of interest as well as the position of the fiber.

Further, it is to be appreciated that the optical fiber 118 may degrade over time, or during use. For example, the optical fiber 118 may be sacrificial and the length can degrade as tissue 126 is cut or as laser energy is emitted from the distal tip 704. As a specific example, the optical fiber 118 may degrade by about 1 mm for every 30 grams of tissue 126 that is morcellated. However, it is noted that fiber degradation rates may vary widely, and the above rate of degradation is given for example only. Consequently, in a treatment where 100 grams of tissue is to be dissected and removed, the total degradation of the optical fiber 118 may be around 5 mm. During such a procedure, the optical fiber 118 would need to be repositioned to maintain the distal tip 704 within the specified range from the central point 604.

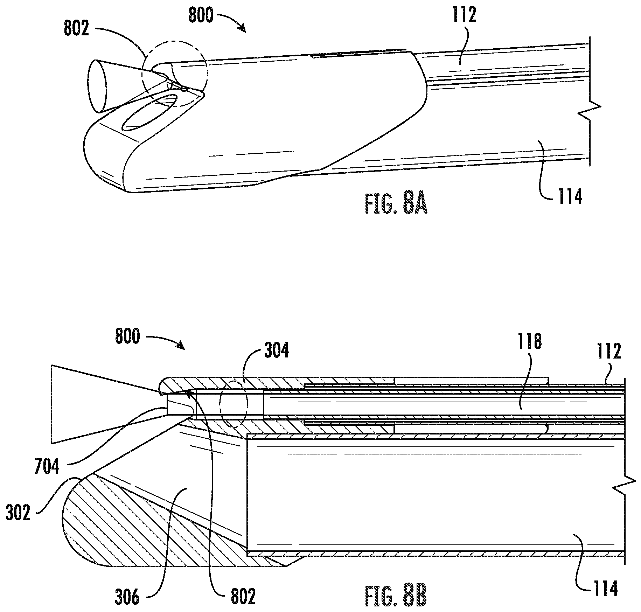

**FIG. 8A** and **FIG. 8B** illustrate a tip 800. The tip 800 can be like the other tips described herein (e.g., the 300, the tip 400a, the tip 400b, the tip 400c, the tip 400d, the tip 500a, the tip 500b, the tip 500c, or the tip 600) or can include features described with respect to these tips and can be provided as the tip 110 of the tissue dissecting apparatus 100 or 200. The tip 800 includes an optical fiber stopper 802 arranged to prevent the optical fiber 118 (or the distal tip 704) from being advanced past a point away from the body of the tip 800. The optical fiber stopper 802 may be provisioned at the circumference of the first aperture 304 and at a top end of the body of the tip 800 such that movement of the optical fiber 118 out of the tip 800 past a certain point can be limited.

With some examples, displacement of the optical fiber 118 into a desired position may be achieved by preloading the optical fiber 118 with a spring 804. The spring 804 may be arranged to mechanically advance the optical fiber 118 automatically from the rebound force of the spring 804. The optical fiber stopper 802 can be arranged to provide a counter or stopping point for the mechanical force of the spring on the optical fiber 118. In such a manner, the optical fiber 118 can be automatically positioned a specified distance away from the body of the tip 800.

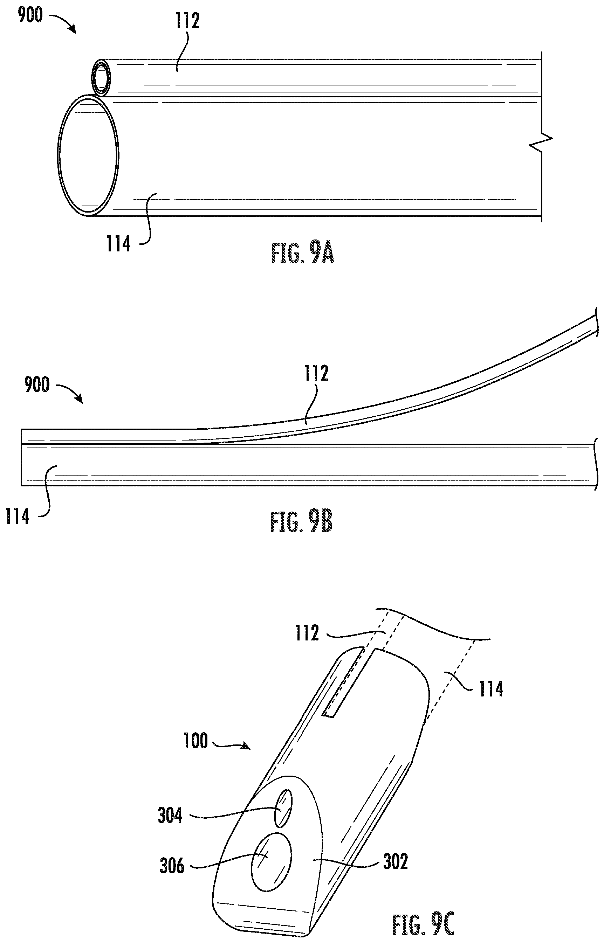

In some embodiments, the fiber tube 112 may be positioned on the suction tube 114 while in other embodiments, the fiber tube 112 may be positioned within the suction tube 114. For example, **FIG. 9A** and **FIG. 9B** illustrate a cannula 900, which can be provided as the cannula 108 of the tissue dissecting apparatus 100 or 200. As can be seen from these figures, cannula 900 comprises the fiber tube 112 is disposed on the suction tube 114. **FIG. 9C** illustrates the tip 100 arranged to encompass or accept the tip of the cannula 900. The aperture 304 of the tip 100 may accommodate the fiber tube 112 and the aperture 306 of the tip 100 may accommodate the suction tube 114 in such a manner that the tip 100 partially extends over the circumference of the cannula 900.

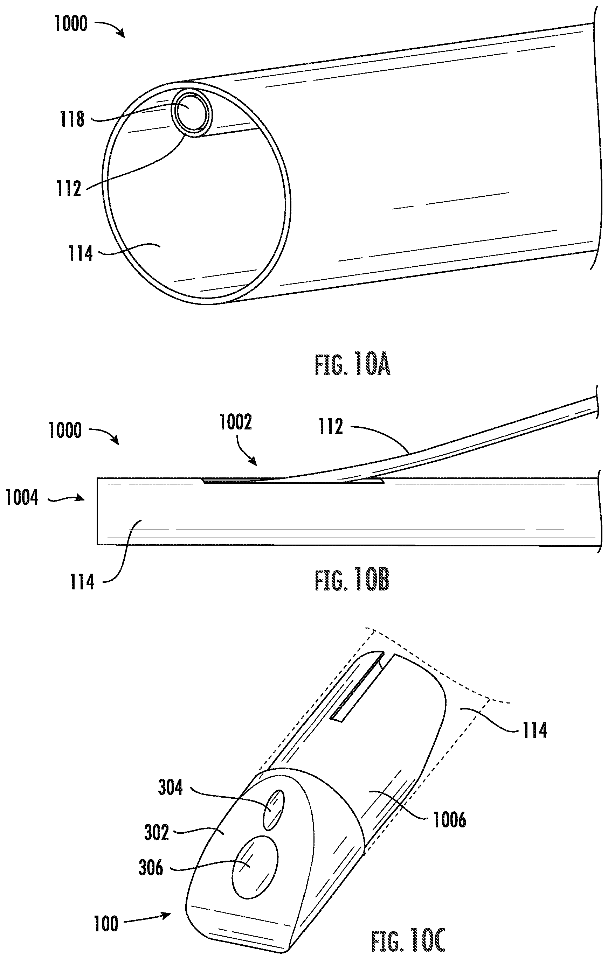

Alternatively, **FIG. 10A** and **FIG. 10B** depict a cannula 1000, which can be provided as the cannula 108 of the tissue dissecting apparatus 100 or 200. As can be seen from this figure, the cannula 1000 comprises the fiber tube 112 disposed inside the suction tube 114 at the distal end proximate to the end of the cannula 1000. The cannula 1000 may comprise a slit 1002 arranged in the suction tube 114 to allow the fiber tube 112 to enter the suction tube 114 somewhere along the length of the suction tube 114 such that the fiber tube 112 is disposed inside the suction tube 114 at the distal end 1004 of the cannula 1000. The cannula 1000 described here provides an advantage in that the suction tube 114 can have a greater diameter, which provides for extraction of larger pieces of tissue and reduces the likelihood of clogging. Further, the cannula itself has a more streamlined outer circumference.

**FIG. 10C** illustrates the tip 100 arranged to with a reduced diameter portion 1006, which is configured to fit within the inner diameter of the suction tube 114. The fiber tuber 112 is arranged to fit within the aperture 304 while the aperture 306 is fluidly coupled to the suction tube 114. Additionally, the tip 100 is arranged such that the distal end (e.g., the end with the inclined tip surface 302 protrudes out of the suction tube 114.

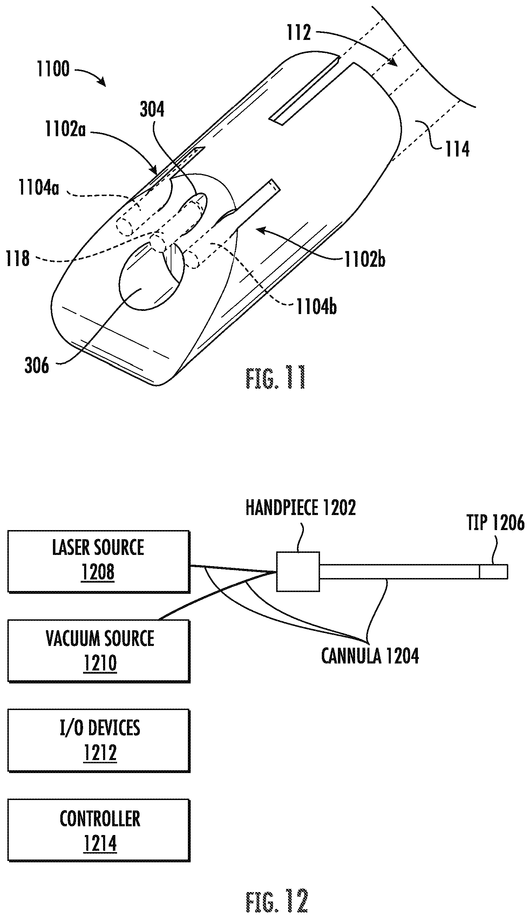

**FIG. 11** illustrates a tip 1100 for a tissue dissecting apparatus. The tip 1100 can be like the other tips described herein (e.g., the 300, the tip 400a, the tip 400b, the tip 400c, the tip 400d, the tip 500a, the tip 500b, the tip 500c, the tip 600, or the tip 800) or can include features described with respect to these tips and can be provided as the tip 110 of the tissue dissecting apparatus 100 or 200. The tip 1100 includes additional apertures 1102a and 1102b arranged to accommodate one or more other tools or devices 1104a and 1104b. For example, apertures 1102a and 1102b can be arranged to accept reference pins for providing an indication of the location of the optical fiber 118 or to accept an image capture device and/or a light source.

In a specific example, the device 1104a can be a light source (e.g., fiber optic light, light emitting diode, or the like) arranged to illuminate a cavity of a body (e.g., a bladder, or the like) while the device 1104b can be an image capture device (e.g., a camera, an optical waveguide, or the like).

**FIG. 12** illustrates a tissue dissecting system 1200 while FIG. 12 illustrates a morcellation and extraction therapy routine 1300. The system 1200 and the routine 1300 are described together for clarity. However, it is to be appreciated that the routine 1300 can be implemented with a tissue dissecting system different than the system 1200. Further, the system 1200 can be used or implemented in a therapy or routine different than the routine 1300.

The tissue dissecting system comprises a handpiece 1202, a cannula 1204, and a tip 1206. In general, the handpiece 1202 can be any handpiece arranged to guide or control placement of the cannula 1204 and the tip 1206. For example, the handpiece 1202 can be the handpiece 102 or the handpiece 202 described above. Likewise, the cannula 1204 can be any of a variety of cannulas comprising a suction tube and an optical fiber, such as, for example, the cannula 108, the cannula 900, or the cannula 1000. Similarly, the tip 1206 can be any of the tips described above, such as, for example, the tip 300, the tip 400a, the tip 400b, the tip 400c, the tip 400d, the tip 500a, the tip 500b, the tip 500c, the tip 600, the tip 800, or the tip 1100.

The tissue dissecting system 1200 further includes a laser source 1208, a vacuum source 1210, input and/or output (I/O) devices 1212, and a controller 1214. The laser source 1208 can be any of a variety of laser sources, such as, for example a solid-state laser, a fiber laser, a gas laser, or the like. In specific examples, the laser source can be a holmium or a thulium gas laser. The laser source 1208 can be optically coupled to an optical fiber (not shown) in the cannula 1204 and activated (via I/O devices 1212, or the like) to cause laser energy to be emitted from the tip 1206 to enucleate, dissect, or morcellate tissue (not shown). Likewise, the vacuum source can be any of a variety of vacuum sources arranged to create a vacuum in the vacuum tube (not shown) of the cannula 1204. The vacuum source 1210 can be fluidly coupled to the vacuum tube of the cannula 1204 and activated (via I/O devices 1212, or the like) to cause the tissue to be extracted from the body of the patient.

The routine 1300 can begin at block 1302 "couple a handpiece and a cannula to a laser source and a vacuum source and affix a tip to the distal end of the cannula" a clinician (e.g., physician, technician, nurse, or the like) can couple the handpiece 1202 and the cannula 1204 to the laser source 1208 and the vacuum source 1210 and can affix the tip 1206 to the distal end of the cannula 1204.

The routine 1300 can continue to block 1304 "insert a cannula and tip of a tissue dissecting system into a body of a patient" a clinician (e.g., physician, technician, nurse, or the like) can insert the cannula 1204 and the tip 1206 into a body of a patient (not shown). It is noted that the present disclosure is often used in conjunction with a nephroscope. In particular, the cannula and the tip are inserted into the working channel of the nephroscope.

The I/O devices 1212 can be any number and type of I/O devices, such as, for example, a foot pedal, a voice activated input device, a keyboard, a mouse, an audible output device, a visual output device, or the like. The laser source 1208 and the vacuum source 1210 can be activated by the I/O devices 1212. Furthermore, the handpiece 1202 can include the optical fiber adjustment unit 120, the position indication unit 122 and the locking unit 124, which can be activated or actuated in combination with the I/O devices 1212 to control operation of the system 1200. As such, the I/O devices 1212 can be disposed on the handpiece 1202 or outside the handpiece 1202. Typically, the system 1200 will include multiple I/O devices where some are provided in combination with the handpiece (e.g., optical fiber adjustment) and other provided outside the handpiece housing (e.g., activation foot pedals, or the like).

Routine 1300 includes block 1306 "position the tip adjacent to tissue, in the body of the patient, to be dissected and extracted" and block 1308 "adjust the position of the optical fiber with respect to the tip" where a clinician can position the cannula 1204 and the tip 1206 within the body of the patient using the handpiece and can adjust the distal end of the optical fiber (e.g., the optical fiber 118) with respect to the tip 1206 using the handpiece 1202 and/or the I/O devices 1212.

Continuing to block 1310 "activate the laser source and the vacuum source to dissect and extract the tissue" the clinician can activate (e.g., via I/O devices 1212, or the like) the laser source 1208 and the vacuum source 1210 to dissect via laser energy emissions from the tip 1206 and extract the dissected tissue via vacuum pressure from the tip 1206. With some examples, the vacuum source 1210 can be activated prior to the laser source 1208 to cause the tissue to be attracted to the tip 1206 (e.g., via vacuum pressure, or the like) or to draw the tissue to within a selected distance to the tip 1206. After which, the laser source 1208 can be activated and the tissue dissected and extracted.

Continuing to decision block 1312 "all tissue extracted" a determination can be made as to whether all desired tissue has been dissected and extracted. From decision block 1312, routine 1300 can return to block 1306 or can end. Where a determination, at decision block 1312, is made that all tissue has been dissected and extracted, routine 1300 can end. Alternatively, where a determination, at decision block 1312, is made that all tissue has not been dissected and extracted, routine 1300 can return to block 1306 where the cannula 1204 and the tip 1206 can be repositioned (e.g., at block 1306), the optical fiber can be readjusted, which may be necessitated due to degradation or the like (e.g., at block 1308) and more tissue can be dissected and extracted (e.g., at bock 1310).

Tissue dissecting system 1200 further includes a controller 1214. The controller 1214 can comprise circuitry, memory devices and instructions executable by circuitry, or a combination or circuitry and memory devices comprising instructions executable by the circuitry. The controller 1214 can be coupled to the laser source 1208, the vacuum source 1210, and the I/O devices 1212 and arranged to control various operating parameters of the sources based on preprogrammed parameters and/or feedback or input received from the I/O devices. Furthermore, the controller 12124 can be arranged to provide feedback regarding operation of the system to a clinician via the I/O devices 1212.

The devices and/or methods disclosed and claimed herein can be made and executed without undue experimentation considering the present disclosure. While the devices and methods of this disclosure have been described in terms of preferred embodiments, it may be apparent to those of skill in the art that variations can be applied to the devices and/or methods and in the steps or in the sequence of steps of the method described herein without departing from the scope of the disclosure. All such similar substitutes and modifications apparent to those skilled in the art are deemed to be within the scope and concept of the disclosure as defined by the appended claims.

## Claims

1. A tip (110; 300) for a tissue dissecting apparatus (100; 200), comprising:
a body comprising a tip surface (302);
a first aperture (304) disposed in the tip surface (302) and defined along a longitudinal axis of the body, wherein the first aperture (304) is configured to accommodate an optical fiber (118); and
a second aperture (306) disposed in the tip surface (302) adjacent the first aperture (304), wherein the second aperture (306) is connectable to a suction tube (114),
wherein, an angle between the longitudinal axis of the first aperture (304) and the tip surface (302) is greater than or equal to 10 degrees or less than or equal to 35 degrees.

2. The tip (110; 300) of claim 1, wherein a diameter of the second aperture (306) is greater than or equal to 1.8 millimeters (mm) and less than or equal to 2.5 mm.

3. The tip (110; 300) of any one of claims 1 or 2, wherein the body comprises tungsten or stainless steel.

4. The tip (110; 300) of any one of claims 1 to 3, wherein the body comprises one or more additional apertures comprising a reference pin.

5. The tip (110; 300) of any one of claims 1 to 4, wherein the body comprises a plurality of additional apertures.

6. The tip (110; 300) of claim 5, wherein the plurality of additional apertures are arranged to accommodate an image capture device and a light source.

7. The tip (110; 300) of any one of claims 1 to 6, wherein a diameter of the first aperture (304) is less than a diameter of the second aperture (306).

8. The tip (110; 300) of any one of claims 1 to 7, wherein the first aperture (304) and the second aperture (306) are separated by a distance greater than or equal to 1 millimeter (mm) and less than or equal to 4 mm.

9. The tip (110; 300) of any one of claims 1 to 8, comprising a shoulder disposed at a circumference of the first aperture (304), the shoulder being arranged to limit movement of the optical fiber.

10. The tip (110; 300) of any one of claims 1 to 9, comprising a cannula (108) comprising the optical fiber (118), a fiber tube (112) configured to accommodate the optical fiber (118), and the suction tube.

11. The tip (110; 300) of any one of claims 1 to 10, wherein the second aperture (306) comprises:
a first portion extending along the longitudinal axis of the body from a first end to a predetermined distance; and
a second portion extending inclinedly from the first portion to the inclined tip surface (302) of the body.

12. The tip (110; 300) of claim 11, wherein a diameter of the second portion is smaller than a diameter of the first portion.

13. The tip (110; 300) of any one of claims 1 to 12, wherein the second aperture (306) is defined with a circular opening on the inclined tip surface (302).

14. A tissue dissecting system (100; 200) for removing tissue from a body of a patient, comprising:
a cannula (108) comprising a fiber tube and a suction tube, the fiber tube being arranged to
accommodate an optical fiber;
a holder defining a housing, the holder being coupled to a first end of the cannula (108); and
a tip (110; 300) according to any one of claims 1 to 13, the tip (110; 300) being coupled to a
second end of the cannula (108).

## Patentansprüche

1. Spitze (110; 300) für eine Gewebedissektionsvorrichtung (100; 200), aufweisend:
einen Körper, der eine Spitzenoberfläche (302) aufweist;
eine erste Öffnung (304), die in der Spitzenoberfläche (302) angeordnet ist und entlang einer Längsachse des Körpers definiert ist, wobei die erste Öffnung (304) dafür konfiguriert ist, eine optische Faser (118) aufzunehmen; und
eine zweite Öffnung (306), die in der Spitzenoberfläche (302) benachbart zur ersten Öffnung (304) angeordnet ist, wobei die zweite Öffnung (306) mit einem Saugrohr (114) verbindbar ist,
wobei ein Winkel zwischen der Längsachse der ersten Öffnung (304) und der Spitzenoberfläche (302) größer oder gleich 10 Grad oder kleiner oder gleich 35 Grad ist.

2. Spitze (110; 300) nach Anspruch 1, wobei ein Durchmesser der zweiten Öffnung (306) größer oder gleich 1,8 Millimeter (mm) und kleiner oder gleich 2,5 mm ist.

3. Spitze (110; 300) nach irgendeinem der Ansprüche 1 oder 2, wobei der Körper Wolfram oder Edelstahl aufweist.

4. Spitze (110; 300) nach irgendeinem der Ansprüche 1 bis 3, wobei der Körper eine oder mehrere zusätzliche Öffnungen aufweist, die einen Referenzstift aufweisen.

5. Spitze (110; 300) nach irgendeinem der Ansprüche 1 bis 4, wobei der Körper eine Mehrzahl zusätzlicher Öffnungen aufweist.

6. Spitze (110; 300) nach Anspruch 5, wobei die Mehrzahl zusätzlicher Öffnungen dazu eingerichtet sind, eine Bildaufnahmevorrichtung und eine Lichtquelle aufzunehmen.

7. Spitze (110; 300) nach irgendeinem der Ansprüche 1 bis 6, wobei ein Durchmesser der ersten Öffnung (304) kleiner als ein Durchmesser der zweiten Öffnung (306) ist.

8. Spitze (110; 300) nach irgendeinem der Ansprüche 1 bis 7, wobei die erste Öffnung (304) und die zweite Öffnung (306) durch einen Abstand größer oder gleich 1 Millimeter (mm) und kleiner oder gleich 4 mm voneinander getrennt sind.

9. Spitze (110; 300) nach irgendeinem der Ansprüche 1 bis 8, aufweisend eine an einem Umfang der ersten Öffnung (304) angeordnete Schulter, wobei die Schulter dazu eingerichtet ist, eine Bewegung der optischen Faser zu begrenzen.

10. Spitze (110; 300) nach irgendeinem der Ansprüche 1 bis 9, aufweisend eine Kanüle (108), welche die optische Faser (118), ein Faserrohr (112), das dafür konfiguriert ist, die optische Faser (118) aufzunehmen, und das Saugrohrumfasst.

11. Spitze (110; 300) nach irgendeinem der Ansprüche 1 bis 10, wobei die zweite Öffnung (306) aufweist:
einen ersten Abschnitt, der sich entlang der Längsachse des Körpers von einem ersten Ende bis zu einem vorbestimmten Abstand erstreckt; und
einen zweiten Abschnitt, der sich schräg vom ersten Abschnitt zur geneigten Spitzenoberfläche (302) des Körpers hin erstreckt.

12. Spitze (110; 300) nach Anspruch 11, wobei ein Durchmesser des zweiten Abschnitts kleiner als ein Durchmesser des ersten Abschnitts ist.

13. Spitze (110; 300) nach irgendeinem der Ansprüche 1 bis 12, wobei die zweite Öffnung (306) mit einer kreisförmigen Mündung auf der geneigten Spitzenoberfläche (302) definiert ist.

14. Gewebedissektionssystem (100; 200) zum Entfernen von Gewebe aus einem Körper eines Patienten, aufweisend:
eine Kanüle (108), die ein Faserrohr und ein Saugrohr umfasst, wobei das Faserrohr dazu eingerichtet ist, eine optische Faser aufzunehmen;
einen Halter, der ein Gehäuse definiert, wobei der Halter mit einem ersten Ende der Kanüle (108) verbunden ist; und
eine Spitze (110; 300) nach irgendeinem der Ansprüche 1 bis 13, wobei die Spitze (110; 300) mit einem zweiten Ende der Kanüle (108) verbunden ist.

## Revendications

1. Pointe (110 ; 300) pour un appareil de dissection de tissu (100 ; 200), comprenant :
un corps comprenant une surface de pointe (302) ;
un premier orifice (304) disposé dans la surface de pointe (302) et défini le long d'un axe longitudinal du corps, dans laquelle le premier orifice (304) est configuré pour recevoir une fibre optique (118) ; et
un deuxième orifice (306) disposé dans la surface de pointe (302) adjacent au deuxième orifice (304), dans laquelle le deuxième orifice (306) peut être relié à un tube d'aspiration (114),
dans laquelle, un angle entre l'axe longitudinal du premier orifice (304) et la surface de pointe (302) est supérieur ou égal à 10 degrés ou inférieur ou égal à 35 degrés.

2. Pointe (110 ; 300) selon la revendication 1, dans laquelle un diamètre du deuxième orifice (306) est supérieur ou égal à 1,8 millimètre (mm) et inférieur ou égal à 2,5 mm.

3. Pointe (110 ; 300) selon l'une quelconque des revendications 1 ou 2, dans laquelle le corps comprend du tungstène ou de l'acier inoxydable.

4. Pointe (110 ; 300) selon l'une quelconque des revendications 1 à 3, dans laquelle le corps comprend un ou plusieurs orifices supplémentaires comprenant une aiguille de référence.

5. Pointe (110 ; 300) selon l'une quelconque des revendications 1 à 4, dans laquelle le corps comprend une pluralité d'orifices supplémentaires.

6. Pointe (110 ; 300) selon la revendication 5, dans laquelle la pluralité d'orifices supplémentaires sont agencés pour contenir un dispositif de capture d'images et une source de lumière.

7. Pointe (110 ; 300) selon l'une quelconque des revendications 1 à 6, dans laquelle un diamètre du premier orifice (304) est inférieur à un diamètre du deuxième orifice (306).

8. Pointe (110 ; 300) selon l'une quelconque des revendications 1 à 7, dans laquelle le premier orifice (304) et le deuxième orifice (306) sont séparés d'une distance supérieure ou égale à 1 millimètre (mm) et inférieure ou égale à 4 mm.

9. Pointe (110 ; 300) selon l'une quelconque des revendications 1 à 8, comprenant un épaulement disposé au niveau d'une circonférence du premier orifice (304), l'épaulement étant agencé pour limiter le déplacement de la fibre optique.

10. Pointe (110 ; 300) selon l'une quelconque des revendications 1 à 9, comprenant une canule (108) comprenant la fibre optique (118), un tube de fibre (112) configuré pour recevoir la fibre optique (118), et le tube d'aspiration.

11. Pointe (110 ; 300) selon l'une quelconque des revendications 1 à 10, dans laquelle le deuxième orifice (306) comprend :
une première partie s'étendant le long de l'axe longitudinal du corps d'une première extrémité à une distance prédéterminée ; et
une deuxième partie s'étendant de manière inclinée de la première partie à la surface de pointe inclinée (302) du corps.

12. Pointe (110 ; 300) selon la revendication 11, dans laquelle un diamètre de la deuxième partie est inférieur à un diamètre de la première partie.

13. Pointe (110 ; 300) selon l'une quelconque des revendications 1 à 12, dans laquelle le deuxième orifice (306) est défini avec une ouverture circulaire sur la surface de pointe inclinée (302).

14. Système de dissection de tissu (100 ; 200) pour retirer du tissu d'un corps d'un patient, comprenant :
une canule (108) comprenant un tube de fibre et un tube d'aspiration, le tube de fibre étant agencé pour recevoir une fibre optique ;
un support définissant un logement, le support étant couplé à une première extrémité de la canule (108) ; et
une pointe (110 ; 300) selon l'une quelconque des revendications 1 à 13, la pointe (110 ; 300) étant couplée à une deuxième extrémité de la canule (108).
